(19) Europäisches Patentamt
European Patent Office
Office européen des brevets

(11) Veröffentlichungsnummer: **0 292 023 A2**

# (12) EUROPÄISCHE PATENTANMELDUNG

(21) Anmeldenummer: **88108271.3**

(22) Anmeldetag: **24.05.88**

(51) Int. Cl.⁴: **C07H 19/06 , A61K 31/70**

Patentansprüche für folgende Vertragsstaaten: ES + GR.

(30) Priorität: **22.05.87 GB 8712115**

(43) Veröffentlichungstag der Anmeldung: **23.11.88 Patentblatt 88/47**

(84) Benannte Vertragsstaaten: **AT BE CH DE ES FR GB GR IT LI LU NL SE**

(71) Anmelder: **F. HOFFMANN-LA ROCHE & CO. Aktiengesellschaft**

**CH-4002 Basel(CH)**

(72) Erfinder: **Machin, Peter James**
**42 Parklands Road**
**London SW16 6TF(GB)**
Erfinder: **Martin, Joseph Armstrong**
**10 The Chownes**
**West Common Harpenden Herts.(GB)**
Erfinder: **Thomas, Gareth John**
**2B Woodland Way**
**Oaklands Welwyn Herts.(GB)**

(74) Vertreter: **Lederer, Franz, Dr. et al**
**Van der Werth, Lederer & Riederer**
**Patentanwälte Lucile-Grahn-Strasse 22**
**D-8000 München 80(DE)**

## (54) Pyrimidinderivate.

(57) Pyrimidinderivate der Formel

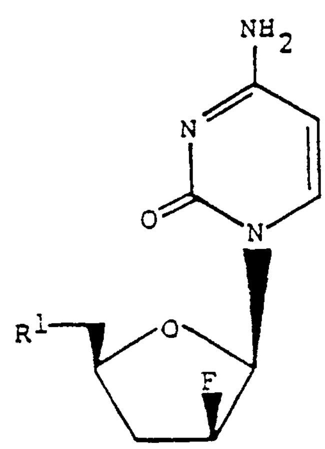

I

worin $R^1$ Hydroxy oder verestertes Hydroxy ist, sowie Amide und Schiff'sche Basen davon haben antivirale Wirksamheit und sind zur Behandlung oder Prophylaxe von viralen, insbesondere retroviralen Infektionen, wie Visna und HIV-Infektionen, verwendbar. Sie können ausgehend von entsprechenden 3′-hydroxylierten Verbindungen hergestellt werden.

EP 0 292 023 A2

## Pyrimidinderivate

Die vorliegende Erfindung betrifft Pyrimidinderivate der Formel

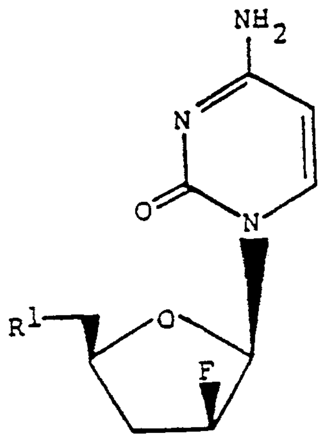

I

worin $R^1$ Hydroxy oder verestertes Hydroxy ist
sowie Amide und Schiff'sche Basen davon.

Diese Verbindungen haben wertvolle pharmakologische Eigenschaften. Insbesondere haben sie antivirale Wirksamkeit und können zur Behandlung oder Prophylaxe von viralen, insbesondere von retroviralen Infektionen, wie Visna und HIV-Infektionen, verwendet werden.

Gegenstand der Erfindung sind die weiter oben definierten Verbindungen an sich sowie zur Verwendung als therapeutisch aktive Substanzen; ein Verfahren zur Herstellung dieser Verbindungen. Medikamente auf der Basis dieser Verbindungen und die Verwendung dieser Verbindungen in der Kontrolle oder Vorbeugung von Krankheiten, insbesondere in der Behandlung oder Prophylaxe von viralen Infektionen oder für die Herstellung von Medikamenten für die Behandlung oder Prophylaxe von viralen Infektionen.

Die veresterten Hydroxygruppen $R^1$ können herkömmliche veresterte Hydroxygruppen sein, z.B. Alkanoyloxygruppen, wie Acetoxy, Propionyloxy oder Butyryloxy; Aroyloxygruppen, wie Benzoyloxy oder substituiertes Benzoyloxy z.B. p-Toluoyloxy oder p-Chlorbenzoyloxy. Amide der Verbindungen der Formel I sind an der 4-Aminogruppe mit einer herkömmlichen Acylgruppe, wie einer Alkanoylgruppe. z.B. Acetyl, Propionyl, Butyryl oder Pivaloyl einer Aroylgruppe, z.B. Benzoyl, oder einer von einer Aminosäure abgeleiteten Acylgruppe. z.B. Glycyl, Alanyl oder Lysyl, gebildet. Schiff'sche Basen der Verbindungen der Formel I werden an der 4-Aminogruppe mit herkömmlichen Aldehyden oder Ketonen, wie Benzaldehyd, oder mit Dimethylformamidacetal gebildet.

Das 1-(2′,3′-Dideoxy-2′-fluor-b-D-arabinofuranosyl)cytosin (im folgenden DFAC) ist eine besonders bevorzugte Verbindung der Formel I.

Nach dem Verfahren der vorliegenden Erfindung werden die oben genannten Verbindungen dadurch hergestellt, dass man

(a) die 3′-Hydroxygruppe aus einer Verbindung der allgemeinen Formel

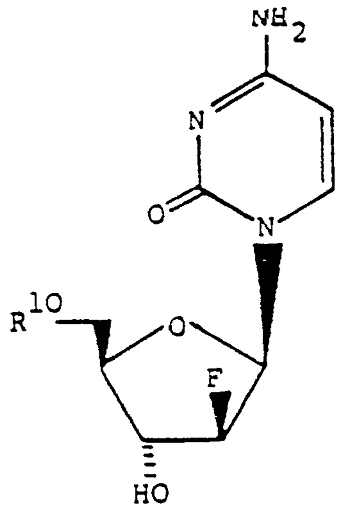

II

worin $R^{10}$ verestertes Hydroxy ist,

abspaltet und gewünschtenfalls im erhaltenen Produkt die veresterte Hydroxygruppe in die Hydroxygruppe umwandelt oder

(b) zur Herstellung einer Verbindung der Formel I, in der $R^1$ verestertes Hydroxy ist, eine Verbindung der Formel I, in der $R^1$ Hydroxy ist, verestert, oder

(c) zur Herstellung eines Amids oder einer Schiff'schen Base einer Verbindung der Formel I, eine erhaltene Verbindung der Formel I in ein Amid oder in eine Schiff'sche Base umwandelt.

Die Abspaltung einer 3′-Hydroxygruppe aus einer Verbindung der Formel II nach der Verfahrensvariante (a) kann man dadurch bewerkstelligen, dass man zuerst eine solche Verbindung der Formel II in den entsprechenden Sulfonsäureester, wie das Mesylat, durch Behandlung mit einem Sulfonsaurehalogenid, wie Methansulfonylchlorid, zweckmässig in Gegenwart eines säurebindenden Mittels, insbesondere eines tertiären Amins, wie Pyridin, und bei niedriger Temperatur, z.B. 0-5°C, umwandelt. Den so erhaltenen Sulfonsäureester kann man dann in das 3′-Jodid in an sich bekannter Weise umwandeln, z.B. durch Behandlung mit einem Alkalimetalljodid, wie Natriumjodid, in einem geeigneten Medium, wie einem aliphatischen Keton. z.B. Aceton oder 2-Butanon, bei erhöhter Temperatur, vorzugsweise bei Rückflusstemperatur des Gemisches. Das entstandene 3′-Jodid kann dann in die erwünschte Verbindung der Formel I, in der $R^1$ verestertes Hydroxy ist, überge führt werden durch Hydrierung in Gegenwart eines Palladiumkatalysators. In einer Variante kann man das 3′-Jodid mit Tributylzinnhydrid in Gegenwart eines freie Radikale erzeugenden Mittels. z.B. Azobisisobutyronitril, zweckmässig in einem inerten organischen Lösungsmittels, wie einem aromatischen Kohlenwasserstoff, z.B. Benzol oder Toluol, und bei erhöhter Temperatur, z.B. etwa 60-90°C, in die erwünschte Verbindung der Formel I, in der $R^1$ verestertes Hydroxy ist, umwandeln.

Eine weitere Methode zur Abspaltung der 3′-Hydroxygruppe aus einer Verbindung der Formel II besteht darin, dass man zunächst eine solche Verbindung mit Phenylthionochlorformiat, zweckmässig in einem inerten organischen Lösungsmittel, wie Acetonitril, in Gegenwart eines säurebindenden Mittels, wie eines tertiären Amins, z.B. Pyridin oder 4-Dimethylaminopyridin, und bei etwa Raumtemperatur zur entsprechenden 3′-O-Phenoxythiocarbonyl-Verbindung umwandelt. Eine solche Verbindung kann man dann in die erwünschte Verbindung der Formel I, in der $R^1$ verestertes Hydroxy ist, durch Behandlung mit Tributylzinnhydrid in Gegenwart eines freie Radikale erzeugenden Mittels in zur weiter oben beschriebenen Methode analoger Weise umwandeln. In einer bevorzugten Ausführungsform dieser Methode wird die Verbindung der Formel II vor der Reaktion mit Phenylthionochlorformiat an der 4-Aminogruppe acyliert. Bei dieser Ausführungsform ist das nach der Behandlung mit Tributylzinnhydrid erhaltene Produkt ein der Verbindung der Formel I, in der $R^1$ verestertes Hydroxy ist, entsprechendes Amid.

Die Umwandlung der veresterten Hydroxygruppe $R^1$ in eine Hydroxygruppe kann man in an sich bekannter Weise durchführen, z.B. durch Behandlung mit einer gesättigten Ammoniaklösung in einem Alkanol, z.B. Methanol. Falls das Produkt eine Acylaminogruppe in 4-Stellung enthält, kann diese Gruppe gleichzeitig in eine Aminogruppe umgewandelt werden.

Eine Verbindung der Formel I, in der $R^1$ Hydroxy ist, kann nach der Verfahrensvariante (b) in eine Verbindung der Formel I, in der $R^1$ verestertes Hydroxy ist, verestert werden. Diese Veresterung kann man in an sich bekannter Weise durchführen, z.B. durch Behandlung des Hydrochlorids einer Verbindung der Formel I mit einem geeigneten Säurehalogenid in einem inerten organischen Lösungsmittel, wie Dimethylformamid.

Die Umwandlung einer Verbindung der Formel I in ein Amid oder eine Schiff'sche Base nach der Vefahrensvariante (c) kann man auch in an sich bekannter Weise durch Behandlung mit einem geeigneten Acylierungsmittel bzw. einem geeigneten Aldehyd, Keton oder Dimethylformamidacetal bewerkstelligen.

Die Ausgangsmaterialien der Formel II kann man z.B. dadurch herstellen, dass man die 3′-Acyloxygruppe in einer Verbindung der Formel

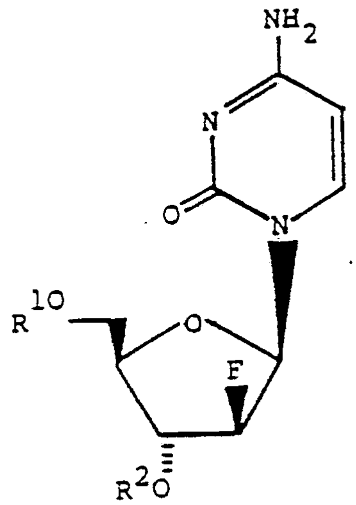

III

worin $R^{10}$ die obige Bedeutung hat und $R^2$ Acyl ist, in eine 3′-Hydroxygruppe umwandelt.

Diese Umwandlunq kann man in an sich bekannter Weise durchführen, z.B. durch Behandlung mit Ammoniak oder einem geeigneten Amin, wie Methylamin, Aethylamin, n-Propylamin oder Triäthylamin, in einem nieder-Alkanol, wie Methanol, zweckmässig bei etwa Raumtemperatur. Durch geeignete Auswahl des Reagenz und der Reaktionsbedingungen kann man vermeiden, dass gleichzeitig die veresterte Hydroxygruppe $R^{10}$ in Hydroxy umgewandelt wird.

Die Verbindungen der Formel III sind bekannt oder Analoga von bekannten Verbindungen und können in Analogie zu letzteren hergestellt werden.

Die in vitro antivirale Aktivität der Pyrimidine der vorliegenden Erfindung kann man wie folgt nachweisen.

(A) Aktivität gegen Schaf-Lentiviren

In diesem Versuch verwendet man Schaf-Lentiviren des Stammes WLC-1, der im Plexus chorioideus (SCP) des Schafs mit 10% fötalem Rinderserum enthaltenden Medium gezüchtet wurde. Die Verbindungen werden in 96 Vertiefungen enthaltenden Platten getestet. Jede Vertiefung enthält $10^4$ SCP-Zellen, die 48 Stunden inkubiert wurden. Die Testverbindungen werden, gelöst in Dimethylsulfoxid (DMSO), bis zu einer Endkonzentration von 1% DMSO hinzugefügt, zusammen mit einem Virus-Inokulum, das innert 12 Tagen einen 100%igen cytopathischen Effekt bewirkt. Nach 12-tägiger Inkubation bei 37° C werden die Platten mit Kristallviolett angefärbt. Effekt in den die Testverbindungen enthaltenden Vertiefungen wird visuell durch Vergleich mit lediglich den Virus enthaltenden Vertiefungen und nicht-infizierten Vertiefungen ermittelt. Die Wirksamkeit (MPC) ist die minimale Konzentration der Testverbindung, die einen 100%igen Schutz bewirkt. In diesem Versuch hat DFAC einen MPC von 2,5 $\mu$M.

(B) Wirksamkeit gegen HIV

In diesem Versuch verwendet man HTLV III (Stamm RF), gezüchtet in C8166-Zellen (eine menschliche $CD4^+$ T-Lymphoblastenlinie), RPM1 1640 Medium mit Bicarbonatpuffer, Antibiotika und 10%igem fötalem Rinderserum. Eine Zellensuspen sion wird mit der 10-fachen $TCD_{50}$ des Virus infiziert. Man lässt 90 Minuten bei 37° C adsorbieren. Die Zellen werden mit dem Medium gewaschen. Der Versuch wird in 6 ml Gewebekulturröhren durchgeführt. Jedes Rohr enthalt 2 x $10^5$ infizierte Zellen in 1,5 ml Medium. Es werden 15 $\mu$l einer Lösung der Substanz in wässrigem Medium oder in DMSO zugesetzt. Nach 72 Stunden

Inkubation bei 37 °C in einer feuchten 5% $CO_2$ enthaltenden Atmosphäre werden die Kulturen zentrifugiert. Ein aliquoter Teil des Ueberstandes wird solubilisiert und einem Antigenabfangversuch unterworfen unter Verwendung eines primären Antiserums mit besonderer Reaktivität gegen virales Protein 24 und eines Meerrettich-Peroxidase-Detektionssystems. Die Farbenerzeugung wird spektrophotometrisch gemessen und in Abhängigkeit der Konzentration der Testsubstanz dargestellt. Die einen 50%igen Schutz erzeugende Konzentration ($IC_{50}$) wird ermittelt. In diesem Test hat DFAC ein $IC_{50}$ von 1 $\mu$M.

Die Pyrimidinderivate der vorliegenden Erfindung können in Form pharmazeutischer Präparate, die diese Verbindungen zusammen mit einem vertraglichen pharmazeutischen Träger enthalten als Medikamente verwendet werden. Diese Präparate können oral, z.B. in Form von Tabletten, Dragées, Hartgelatinekapseln, Weichkapseln, Lösungen, Emulsionen oder Suspensionen, oder parenteral, z.B. in Form von Injektionslösungen, verabreicht werden.

Die weiter oben erwähnten Träger können pharmazeutisch inerte, anorganische oder organische Materialien sein. Beispiele von Träger für Tabletten, Dragées und Hartkapseln sind Lactose, Maisstärke oder Derivate davon, Talk, Stearinsäure oder Salze davon. Beispiele von Träger für Weichkapseln sind pflanzliche Oele, Wachse, Fette, halbfeste und flüssige Polyole. Beispiele von Träger für die Herstellung von Lösungen oder Sirups sind Wasser, Polyole, Saccharose, Invertzucker und Glucose. Beispiele von Träger für Injektionslösungen sind Wasser, Alkohole, Polyole, Glycerol und pflanzliche Oele.

Die pharmazeutischen Präparate können auch herkömmliche pharmazeutische Hilfsmittel enthalten, wie Konservierungs-, Solubilisierungs-, Stabilisierungs-, Befeuchtungsmittel, Emulgatoren, Süssmittel, Farb- oder Riechstoffe, Salze zur Veränderung des osmotischen Druckes, Puffer, Ueberzugsmittel oder Antioxidantien. Sie können auch andere therapeutisch wertvolle Substanzen enthalten.

Die Dosierung, in der die Pyrimidinderivate der vorliegenden Erfindung verabreicht werden können, kann nach oben und unten in Abhängigkeit von Faktoren, wie der einzelnen zu verabreichenden Verbindung, der Schwere der zu behandelnden Erkrankung und dem Zustand des Patienten, variiert werden. Die Pyrimidinderivate der vorliegenden Erfindung kann man in einem täglichen Dosierungsbereich von etwa 0,1 bis 20, vorzugsweise 0,2 bis 15, insbesondere etwa 0,4 bis 10 mg/kg Körpergewicht verabreichen.

Diese Pyrimidinderivate können in Einzeldosen oder vorzugsweise in mehreren Dosen, z.B. bis zu 6 über den Tag verabreicht werden. Eine Einzeldosis für eine solche Verabreichungsform kann z.B. von etwa 0,5 bis 300, vorzugsweise etwa 1,0 bis 300 und insbesondere etwa 2,0 bis 200 mg Pyrimidinderivat enthalten.

Die pharmazeutischen Präparate kann man so herstellen, dass man die obigen Pyrimidinderivate, gewünschenfalls zusammen mit anderen therapeutisch wertvollen Substanzen, mit einem verträglichen pharmazeutischen Träger und gewünschtenfalls einem pharmazeutischen Hilfsstoff vermischt und das Gemisch in die gewünschte Verabreichungsform bringt.

## Beispiel 1

a) Eine Lösung enthaltend 0.94 g 1-(3′-O-Acetyl-5′-O-benzoyl-2′-deoxy -2′-fluor-β-D-arabinofuranosyl)cytosin in 4,9 ml einer 2M Lösung von Ammoniak in Methanol wird 2 Stunden gerührt. Die Lösung wird zur Trockene eingedampft und der Rückstand wird aus Aethylacetat umkristallisiert. Man erhält 0.64 g 1-(5′-O-Benzoyl-2′-deoxy-2′-fluor-β-D-arabinofuranosyl)cytosin, Smp. 135-140 °C.

b) Einer Lösung von 0,4 g des Produkts von Beispiel 1a) in 5,6 ml trockenem Pyridin werden 0,26 g Methansulfonylchlorid bei 0 °C unter Rühren zugetropft. Das Gemisch wird 20 Stunden bei 0-5 °C gerührt und dann mit 0,1 ml Wasser behandelt. Nach 1-stündigem Rühren wird das Gemisch in 20 ml Eis/Wasser geschüttet und mit Aethylacetat extrahiert. Die Aethylacetatextrakte werden mit gesättigter Natriumchloridlösung gewaschen, getrocknet und über Magnesiumsulfat eingedampft. Man erhält 0,5 g 1-(5′-O-Benzoyl-2′-deoxy-2′-fluor -3′-O-methylsulfonyl-β-D-arabinofuranosyl)cytosin.

c) Eine Lösung von 0,5 g des Produkts von Beispiel 1b) und 0,9 g Natriumjodid in 12 ml trockenem 2-Butanon wird unter Rühren 16 Stunden unter Rückfluss erhitzt. Die entstandene Suspension wird zur Trockene eingedampft und der Rückstand wird zwischen Wasser und Aethylacetat aufgetrennt. Die Schichten werden getrennt und die wässrige Schicht wird mit Aethylacetat extrahiert. Die Aethylacetatlösungen werden über Magnesiumsulfat getrocknet und eingedampft. Der Rückstand wird über Silicagel mit Methanol/Dichlormethan (1:9) chromatographiert. Man erhält 0,2 g 1-(5′-O-Benzoyl-2′,3′-dideoxy-2′-fluor -3′-jod-β-D-arabinofuranoyl)cytosin.

d) Eine Lösung von 0,2 g des Produkts von Beispiel 1c) in 8 ml Aethanol wird mit einer Lösung von 0,04 g Natriumbicarbonat in 2 ml Wasser behandelt und dann in Gegenwart von 0,06g eines 10%igen Pd/C-Katalysators 24 Stunden hydriert. Der Katalysator wird abfiltriert und das Filtrat wird zur Trockene eingedampft. Der Rückstand wird zwischen Wasser und Aethylacetat aufgetrennt. Die wässrige Phase wird mit Aethylacetat rückextrahiert. Die Aethylacetatlösungen werden über Magnesiumsulfat getrocknet und eingedampft. Man erhält 0,14 g 1-(5′-O-Benzoyl-2′,3′-dideoxy -2′-fluor-β-D-arabinofuranosyl)cytosin.

e) 0,05 g des Produkts von Beispiel 1d) werden in 2 ml gesättigter Ammoniaklösung in Methanol gelöst. Die Lösung wird 3 Tage stehengelassen und dann zur Trockene eingedampft. Der Rückstand wird in 2 ml Wasser gelöst und die Lösung wird mit Aethylacetat gewaschen. Die wässrige Lösung wird 24 Stunden lyophilisiert. Man erhält 0,02 g DFAC, MS: m/e 229 $(M)^+$, 151, 112.

## Beispiel 2

a) Eine Lösung enthaltend 11,8 g des Produkts von Beispiel 1a) in 1180 ml Methanol wird gerührt und unter Rückfluss erhitzt. Die Lösung wird mit 12 ml Acetanhydrid behandelt und dann viermal innerhalb von 4 Stunden mit je 12 ml Acetanhydrid behandelt. Nach 6 Stunden wird die Lösung zur Trockene eingedampft und der Rückstand wird zusammen mit 300 ml Toluol eingedampft, dann in 1 l Methanol gelöst und dreimal mit je 12 ml Acetanhydrid behandelt. Die Lösung wird zur Trockene eingedampft und der Rückstand wird in Dichlormethan gelöst. Die Lösung wird mit Wasser, gesättigter Natriumbicarbonatlösung und Wasser gewaschen, dann über Natriumsulfat getrocknet und eingedampft. Der Rückstand wird im Vakuum getrocknet. Man erhalt 12,5 g N-Acetyl-1-(5′-O-benzoyl-2′-deoxy -2′-fluor-β-D-arabinofuranosyl)-cytosin.

b) Einer auf 5°C abgekühlten Lösung von 10,7 g des Produkts von Beispiel 2a) und 29,7 g 4-(Dimethylamino)pyridin in 295 ml Acetonitril werden 5,61 g Phenylchlorthionocarbonat unter Argon unter Rühren zugetropft. Das Gemisch wird 10 Minuten bei 5°C gerührt und dann 3 Stunden bei Raumtemperatur. Die entstandene Lösung wird zur Trockene eingedampft und der Rückstand wird in Dichlormethan gelöst. Die Lösung wird mit eiskaltem Wasser, 1M Salzsäure, Wasser, gesättigter Natriumbicarbonatlösung und gesättigter Natriumchloridlösung gewaschen, dann über Natriumsulfat getrocknet und eingedampft. Der Rückstand wird im Vakuum getrocknet. Man erhält 14,2 g N-Acetyl-1-(5′-O-benzoyl-2′-deoxy-2′-fluor-3′-O-phenoxythiocarbonyl-β-D-arabinofuranosyl)cytosin.

c) Einer Lösung von 14,1 g des Produkts von Beispiel 2b) und 0,5 g Azobisisobutyronitril in 520 ml trockenem Toluol werden 11,28 g Tributylzinnhydrid unter Einblasen von Argon und Rühren zugesetzt. Die Zufuhr von Argon wird 30 Minuten fortgesetzt. Das Gemisch wird dann 3 Stunden unter Argon bei 75°C erhitzt. Die Lösung wird zur Trockene eingedampft und der Rückstand wird mit Hexan vermischt. Das Produkt wird abfiltriert und durch Chromatographie auf Silicagel mit Methanol/Dichlormethan (1:9) gereinigt. Man erhält 6,95 g N-Acetyl-1-(5′ -O-benzoyl-2′,3′-dideoxy -2′-fluor-β-D-arabinofuranosyl)cytosin, Smp. 191-193,5°C nach Umkristallisation aus Toluol.

d) Bei 0°C mit Ammoniak gesättigtes Methanol enthaltend 0,5 g des Produkts von Beispiel 2c) werden 3 Stunden gerührt. Die Lösung wird zur Trockene eingedampft und der Rückstand wird in 30 ml Wasser gelöst. Die wässrige Lösung wird mit Aethylacetat gewaschen und dann zur Trockene eingedampft. Der Rückstand wird aus Aethanol umkristallisiert. Man erhält 0,18 g DFAC, Smp. 204-207°C. Die Mutterlaugen der Umkristallisation werden eingedampft und der Rückstand wird in Wasser gelöst. Die wässrige Lösung wird lyophilisiert und der Rückstand wird aus Aethanol umkristallisiert. Man erhält 0,06 g DFAC, Smp. 199-203°C.

Die folgenden Beispiele illustrieren pharmazeutische Zubereitungen mit einem Pyrimidinderivat nach der vorliegenden Erfindung:

## Beispiel A

Tabletten und Kapseln mit folgenden Inhaltsstoffen können in an sich bekannter Weise hergestellt werden:

| Innaltsstotte | | Pro Tablette |
|---|---|---|
| Wirkstoff | | 10 mg |
| Lactose | | 20 mg |
| Stärke | | 4 mg |
| Polyvinylpyrrolidon | | 0,5 mg |
| Magnesiumsteaat | | 0,5 mg |
| | Tablettengewicht | 35 mg |

| Inhaltsstoffe | | Pro Kapsel |
|---|---|---|
| Wirkstoff | | 25 mg |
| Lactose | | 15 mg |
| Natriumstärkeglycollat | | 2,5 mg |
| Magnesiumstearat | | 0,5 mg |
| | Kapselfüllstoff | 43 mg |

## Ansprüche

1. Verbindungen der allgemeinen Formel

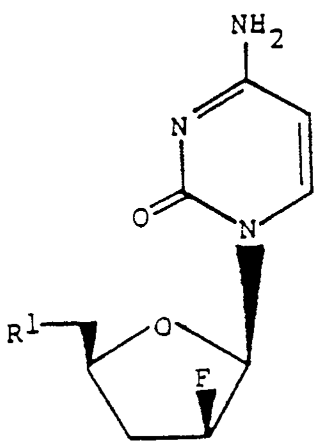

I

worin $R^1$ Hydroxy oder verestertes Hydroxy ist, sowie Amide und Schiff'sche Basen davon.

2. 1-(2′,3′-Dideoxy-2′-fluor-β-D-arabinofuranosyl)cytosin.

3. Ein Pyrimidinderivat nach Anspruch 1 oder 2 zur Verwendung als Heilmittel, insbesondere als Mittel zur Behandlung oder Prophylaxe von viralen Infektionen, insbesondere von retroviralen Infektionen wie HIV-Infektionen.

4. Verfahren zur Herstellung der Pyrimidinderivate nach Anspruch 1, dadurch gekennzeichnet, dass man

(a) die 3′-Hydroxygruppe aus einer Verbindung der allgemeinen Formel

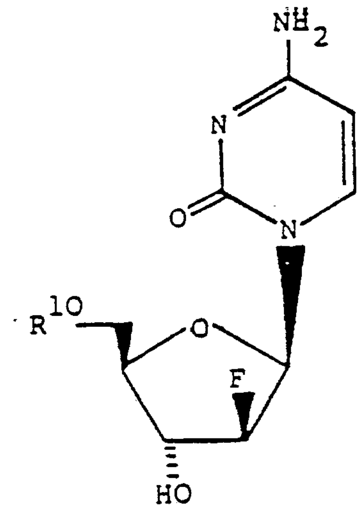

II

worin $R^{10}$ verestertes Hydroxy ist,
abspaltet und gewünschtenfalls im erhaltenen Produkt die veresterte Hydroxygruppe in die Hydroxygruppe umwandelt oder

(b) zur Herstellung einer Verbindung der Formel I, in der $R^1$ verestertes Hydroxy ist, eine Verbindung der Formel I, in der $R^1$ Hydroxy ist, verestert oder

(c) zur Herstellung eines Amids oder einer Schiff'schen Base einer Verbindung der Formel I, eine erhaltene Verbindung der Formel I in ein Amid oder in eine Schiff'sche Base umwandelt.

5. Pharmazeutische Präparate insbesondere für die Behandlung oder Prophylaxe von viralen Infektionen, insbesondere von retroviralen Infektionen, wie HIV-Infektionen, enthaltend ein Pyrimidinderivat nach Anspruch 1 oder 2 und ein pharmazeutisches Trägermaterial.

6. Verwendung eines Pyrimidinderivats nach Anspruch 1 oder 2 zur Herstellung von Arzneimitteln, zur Behandlung oder Prophylaxe von viralen Infektionen, insbesondere retroviralen Infektionen, wie HIV-Infektionen.

Patentansprüche für folgende Vertragsstaaten: ES, GR

1. Verfahren zur Herstellung von Verbindungen der allgemeinen Formel

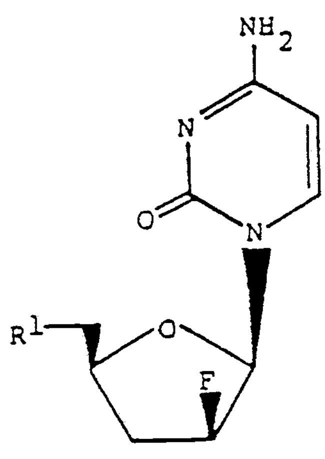

I

worin $R^1$ Hydroxy oder verestertes Hydroxy ist,
von Amiden und Schiff'sche Basen davon, dadurch gekennzeichnet, dass man

(a) die 3′-Hydroxygruppe aus einer Verbindung der allgemeinen Formel

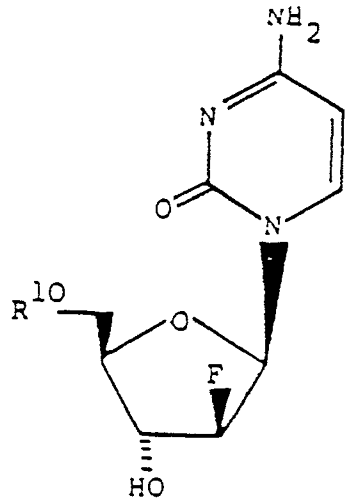

II

worin $R^{10}$ verestertes Hydroxy ist,

abspaltet und gewünschtenfalls im erhaltenen Produkt die veresterte Hydroxygruppe in die Hydroxygruppe umwandelt oder

(b) zur Herstellung einer Verbindung der Formel I, in der $R^1$ verestertes Hydroxy ist, eine Verbindung der Formel I, in der $R^1$ Hydroxy ist, verestert, oder

(c) zur Herstellung eines Amids oder einer Schiff'schen Base einer Verbindung der Formel I, eine erhaltene Verbindung der Formel I in ein Amid oder in eine Schiff'sche Base unwandelt.

2. Verfahren nach Anspruch 1 zur Herstellung von 1-(2′,3′-Dideoxy-2′-fluor-β-D-arabinofuranosyl)-cytosin.

3. Verfahren zur Herstellung pharmazeutischer Präparate, insbesondere für die Behandlung oder Prophylaxe von viralen Infektionen, insbesondere von retroviralen Infektionen, wie HIV-Infektionen, dadurch gekennzeichnet, dass man eine Verbindung der Formel I nach Anspruch 1 oder 2 oder ein Amid oder eine Schiff'sche Base davon, in eine galenische Verabreichungsform bringt.

4. Verwendung einer Verbindung der Formel I nach Anspruch 1 oder 2 eines Amids oder einer Schiff'schen Base davon bei der Herstellung von Arzneimitteln zur Behandlung oder Prophylaxe von viralen Infektionen, insbesondere retroviralen Infektionen, wie HIV-Infektionen.